# EUROPEAN PATENT SPECIFICATION

(11) **EP 4 121 092 B1**
(45) Date of publication and mention of the grant of the patent: **24.04.2024**
(21) Application number: 21711947.8
(22) Date of filing: 16.03.2021
(51) Int. Cl.: A61K 38/21, A61P 31/14, C07K 14/56, C07K 14/44

(54) **HYBRID INTERFERONS FOR TREATING VIRAL INFECTIONS**
HYBRID-INTERFERONE ZUR BEHANDLUNG VON VIRALEN INFEKTIONEN
INTERFERONS HYBRIDES POUR LE TRAITEMENT D'INFECTIONS VIRALES

(30) Priority: 16.03.2020 GB 202003812; 26.05.2020 US 202063030074 P; 19.08.2020 GB 202012967; 20.08.2020 US 202063067930 P; 17.02.2021 GB 202102261
(43) Date of publication of application: 25.01.2023
(73) Proprietor: ILC Therapeutics Ltd, Newhouse Lanarkshire ML1 5UH (GB)
(72) Inventor: STIMSON, William, Lanarkshire, Central Scotland ML1 5UH (GB)
(74) Representative: Murgitroyd & Company
(86) International application number: PCT/GB2021/050649
(87) International publication number: WO 2021/186162

(56) References cited:
- WO-A1-2017/046583
- WO-A2-2006/111745
- WO-A2-2015/136287
- LI GUANGDI ET AL: "Therapeutic options for the 2019 novel coronavirus (2019-nCoV)", NATURE REVIEWS DRUG DISCOVERY, NATURE PUBLISHING GROUP, GB, vol. 19, no. 3, 10 February 2020 (2020-02-10), pages 149-150, XP037050085, ISSN: 1474-1776, DOI: 10.1038/D41573-020-00016-0 [retrieved on 2020-02-10]

## Description

### Field of the invention

The present invention relates to compositions and methods for preventing or treating a viral infection, suitably reduction of viral activity, such as cytopathic effect or plaque formation, in particular coronavirus infection. The invention extends to compositions for preventing or treating infection from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) and Severe Acute Respiratory Syndrome Coronavirus-2 (SARS-CoV-2 or Covid19). The invention further extends to compositions and the use of the compositions of the invention for the treatment and/or prophylaxis of a coronavirus infection for Human and Veterinary therapies.

### Background to the Invention

Coronaviruses (CoV) are a large family of viruses that cause diseases in mammals and birds. In humans, coronaviruses cause respiratory tract infections ranging from the common cold to more severe diseases such as Severe Acute Respiratory Syndrome (SARS), Human Coronavirus NL63, Human Coronavirus HKU1, Middle East Respiratory Syndrome (MERS) and Severe Acute Respiratory Syndrome Coronavirus-2 (SARS-CoV-2 or Covid19). Coronaviruses belong to a family (*Coronaviridae*) of single-stranded RNA viruses that have a lipid envelope studded with club-shaped projections. Coronavirus infection can cause severe disease of the respiratory and enteric systems.

The SARS human coronavirus (SARS-CoV-1) was the first coronavirus to regularly cause severe disease in humans. Middle East Respiratory Syndrome Coronavirus (MERS-CoV) and Severe Acute Respiratory Syndrome Coronavirus-2 (SARS-CoV-2 or Covid19) can also cause severe disease in humans. SARS-CoV-2 (Covid19) has become the most infectious of the human coronaviruses as it can be easily passed from person to person. Symptoms of SARS-CoV-2 include cough, fever and shortness of breath. These viruses can cause severe pneumonia-like symptoms in those infected, with mortality occurring in the most severe cases.

Various anti-viral treatments have been administered to humans infected with SARS-CoV-1 and SARS-CoV-2, including general anti-virals, treatments which inhibit viral cell entry or replication, and immunostimulants. Ribavirin is a broad spectrum anti-viral agent based on a purine nucleoside analogue and is the standard treatment regimen for Hepatitis C. Ribavirin is known to be active against various RNA viruses and is known to show anti-viral activity against animal coronaviruses. However, *in vitro* tests of the efficacy of the drug against SARS-CoV-1 produced a series of negative results and adverse reactions were also been reported.

There are no specific treatments for SARS-CoV-2. Researchers are currently considering the effectiveness of anti-viral drugs, such as Favilavir, Remdesivir, Lopinavir and Ritonavir, against coronaviruses. However, a proven treatment for SARS-CoV-2 has yet to be elucidated.

It is known that different pathogens induce different Interferon-alpha (IFN-α) subtypes in *vitro* and that IFN-α subtypes have different anti-viral, anti-proliferative and immunomodulatory activities. The mechanisms of actions of IFN-α, and in particular individual IFN-α subtypes, are still only partly understood. Infection via a variety of routes has been shown to induce different subtype profiles. IFN-α subtypes bind to the same receptors, activate common signaling pathways and had been expected to have similar immunological functions. All IFN-α subtypes have anti-viral activities, by definition, although their absolute efficacy in this context may vary considerably. In addition, many other biological properties have been described, but with varying potencies, including immunomodulatory and anti-proliferative activities. The pleiotropic effects appear to be due to differential interaction with the receptor chains and signaling through different intracellular pathways to an array of effector molecules. The Type IIFN receptor consists of two chains, IFNR1 and IFNR2. There is a range of binding affinities for each of the 12 IFN-α subtypes with the different receptor chains. IFNα-14 has one of the highest affinities for both of the two interferon receptors, which is why it is so active compared to the other 11 subtypes.

Recombinant interferons, which consist of only the IFN alpha 2 subtype, currently dominate the market for anti-viral indications. There are two main recombinant alpha IFN products, Intron A^{™} from Schering Plough (IFN-alpha 2b) and Roferon^{™} (IFN-alpha 2a) from Roche. In contrast to these single-subtype products, there are several alpha IFN preparations that consist of a mixture of different subtypes. These multi-subtype IFN alpha products are produced either by human leukocytes in response to a stimulation from a virus (such as Multiferon^{™} from Viragen, Inc or its subsidiaries, or Alferon-N^{™} from Interferon Sciences/Hemispherix), or in human lymphoblastoid cells, cultured from a patient with Burkitt's lymphoma (such as Sumiferon^{™} from Sumitomo).

There are currently no completely effective therapeutic or prophylactic treatments for humans infected with a coronavirus and in particular with SARS-CoV-1, MERS-CoV or SARS-CoV-2. There thus is a significant unmet need for a safe, effective treatment for coronaviral infection in humans, and in particular for the treatment and/or prophylaxis of SARS, MERS and SARS-CoV-2.

WO2006/111745 discloses the use of interferon alpha 14 in the treatment of viral disease.

WO2015/136287 and WO2017/046583 disclose interferon subtypes IFN-α10 and IFN-α14 and hybrids thereof for the treatment of a condition where an enhancement of a Thlmediated immune response and suppression of a Th2/Thl7-mediated immune response are desired.

### Summary of Invention

The present inventor submits that it would be desirable to develop a treatment for viral infection, in particular coronaviral infection in humans, and in particular for the treatment and/or prophylaxis of SARS, MERS and SARS-CoV-2. The invention extends to compositions for preventing or treating infection from coronaviral infections by inducing NK and T cell responsiveness. The invention further extends to compositions and the use of the compositions of the invention for the treatment and/or prophylaxis of a coronaviral infection for Human and Veterinary therapies.

The present invention relates to compositions and provides methods for preventing or treating conditions caused as a result of viral infection, in particular coronavirus infection such as from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) and Severe Acute Respiratory Syndrome Coronavirus-2 (SARS-CoV-2 or Covid19). The invention further provides to the use of the compositions of the invention in the treatment and/or prophylaxis of SARS, MERS or SARS-CoV-2. The invention further extends to administering a therapeutically useful amount of the interferon of the invention to a subject in need of treatment along with a therapeutically useful amount of a suitable anti-viral compound.

Suitably the present invention may be used for the treatment of viral conditions such as respiratory viral disease, gastrointestinal viral disease, exanthematous viral disease, hepatic viral disease, cutaneous viral disease, haemorrhagic viral disease, neurological viral disease in particular, Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) and Severe Acute Respiratory Syndrome Coronavirus-2 (SARS-CoV-2 or Covid19). In the absence of vaccines targeted against specific viruses, particularly newly emerging viruses, an effective antiviral drug would be particularly advantageous, suitably in relation to outbreaks of SARs, influenza (particularly H5N1), Zika virus, WNV and EBOV. Suitably the present invention may be used in the treatment of RSV (Respiratory syncytial virus). (not claimed).

Following extensive experimentation, the inventor of the present invention has surprisingly discovered that administering IFN-α14, for example SEQ ID NO:1 or a variant or fragment thereof, HYBRID 1, for example SEQ ID NO:2 or a variant or fragment thereof or HYBRID 2, for example SEQ ID NO:3 or a fragment thereof as described herein results in the suppression or inhibition of the effects of coronavirus infection. The inventor unexpectedly determined that IFN-α14, HYBRID 1 or HYBRID 2 can directly inhibit the cytopathic effect resulting from coronavirus infection. The cytopathic effect or cytopathogenic effect refers to structural changes in host cells that are caused by viral invasion. The inventor demonstrated that IFN-α14, HYBRID 1 or HYBRID 2 also inhibit plaque formation relating to coronavirus infection. Surprisingly, this effect is demonstrated when IFN-α14, HYBRID 1 or HYBRID 2 are administered sublingually. The inventor unexpectedly determined that HYBRID 2 can directly inhibit the cytopathic effect resulting from viral infection. The cytopathic effect or cytopathogenic effect refers to structural changes in host cells that are caused by viral invasion. The inventor demonstrated that HYBRID 2 also inhibit plaque formation relating to viral infection. The activities of HYBRID 2 appear particularly suited to provide antiviral activity.

The present inventor has examined the ability of the synthetic alpha-interferon, Interferon-alpha-14 (SEQ ID NO:1) to inhibit the cytopathic effect and plaque formation caused as a result of infection with SARS-CoV-1 and SARS-CoV-2. The inventor has examined the inhibition of the cytopathic effect in comparison with the anti-viral compound Ribavirin and the multi-subtype interferon Multiferon^{™}. Surprisingly Interferon-alpha14 (SEQ ID NO:1) is active against SARS-CoV-1 and SARS-CoV-2 while Ribavirin and the multi-subtype interferon Multiferon^{™} show much less inhibitory activity.

It is considered that for the treatment of SARS-CoV-2 (Covid19) using IFN-α14 (SEQ ID NO:1), HYBRID 1 (SEQ ID NO:2) (not claimed) or HYBRID 2 (SEQ ID NO:3) or a fragment thereof, the mode of delivery will likely be by direct injection or by aerosol directly into the lungs. Suitably doses in the range 10⁴ to 10⁷ IU per administration may be used. There may also be some relevance for the use of IFN-α14 (SEQ ID NO:1), HYBRID 1 (SEQ ID NO:2) or HYBRID 2 (SEQ ID NO:3) as a sublingual treatment. The inventor has surprisingly discovered that administration of IFN-α14, HYBRID 1 or HYBRID 2 in particular SEQ ID NO:1, 2 or 3 or a fragment thereof, as a sublingual treatment can result in a greater reduction or inhibition of coronavirus activity compared to previous anti-viral medications. In addition, the inventors have determined that very low doses of IFN-α14, HYBRID 1 or HYBRID 2 for example up to 10⁴ to 5×10⁶ IU per day may be used.

This has led to the identification by the inventor of improved therapeutic compositions which have utility in the treatment and/or prophylaxis of coronavirus infection and in particular infection from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) and Severe Acute Respiratory Syndrome Coronavirus-2 (SARS-CoV-2 or Covid19).

The invention is as defined in claims 1 to 7.

Provided herein is a method for the treatment and/or prophylaxis of coronavirus infection, said method comprising the step of:
(i) administering to a subject in need thereof a therapeutically effective amount of an interferon alpha subtype, wherein the interferon alpha subtype is IFN-α14, HYBRID 1, (not claimed) HYBRID 2 or a combination of any of IFN-α14, HYBRID 1 and HYBRID 2, for example of IFN-α14 and HYBRID 1 (not claimed) or IFN-α14 and HYBRID 2 or HYBRID 1 and HYBRID

The coronavirus is Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19).

Tthe coronavirus is Severe Acute Respiratory Syndrome Coronavirus-1 (SARS-CoV-1).

The coronavirus is Severe Acute Respiratory Syndrome Coronavirus-2 (SARS-CoV-2 or Covid19).

Suitably the virus may be selected from a virus which encodes a protein known to antagonise the type 1 IFN response:
Middle East respiratory syndrome coronavirus (MERS-CoV)
Severe acute respiratory syndrome coronavirus (SARS-CoV)
Suitably the virus may be selected from a viral disease caused by an enveloped virus, preferably selected from the group consisting of:
SARS Coronavirus.

The viral infection is selected from coronavirus infection and in particular infection from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) and Severe Acute Respiratory Syndrome Coronavirus-2 (SARS-CoV-2 or Covid19).

The interferon alpha subtype HYBRID 2 comprises or consists of an amino acid sequence SEQ ID NO:3 or a functionally active fragment thereof. Functional activity can be determined by characterisation of the anti-viral activity by use of assays as described herein, for example by one of more of cytopathic effect, plaque formation, effect on production of IL 17F effect on production of CXCL 10 from human leucocytes, effect on interferon gamma production, effect on TNF alpha secretion, induction of apoptosis, effect on NK cell clustering. Functional variants and fragments can be compared to the activity of HYBRID 2 to determine those variants which show the similar activity, suitably a similar range of activities for several assays.

The interferon alpha subtype IFN-α14 comprises or consists of an amino acid sequence SEQ ID NO:1 or a functionally active fragment or variant thereof.

The interferon alpha subtype HYBRID 1 comprises or consists of an amino acid sequence SEQ ID NO:2 or a functionally active fragment or variant thereof.

The interferon alpha subtype HYBRID 2 comprises or consists of an amino acid sequence SEQ ID NO:3 or a functionally active fragment variant thereof.

The method of administration is sublingual administration. In embodiments, (not claimed) the method of administration is oral administration. In embodiments, the method of administration is injection. In embodiments the method of administration is by aerosol delivery to the lungs. The interferon alpha subtype HYBRID 2 is administered by sublingual administration. In embodiments, the interferon alpha subtype can be administered once a day, twice a day, three times a day or four times a day. Suitably, in sublingual administration, a single dose may be provided each day.

The therapeutically effective amount of the interferon alpha subtype IFN-α14, HYBRID 1 and HYBRID 2 is a low dose. In embodiments, the therapeutically effective amount of the interferon alpha subtype IFN-α14, HYBRID 1 and HYBRID 2 is between 10⁴ to 5×10⁶ IU units/ml. In embodiments, the therapeutically effective amount of the interferon alpha subtype IFN-α14, HYBRID 1 and HYBRID 2 is lower than current systemic treatments for coronavirus infection.

The interferon alpha subtype IFN-α14, HYBRID 1 and HYBRID 2 is administered in a dose of 5IU/ml, 10IU/ml, 50IU/ml, 1×10²1U/ml, 1×10³IU/ml, 1×10⁴IU/ml, 1×10⁵IU/ml, 1×10⁶IU/ml or 1×10⁷IU/ml.

The interferon alpha subtype IFN-α14, HYBRID 1 and HYBRID 2 is administered in a dose of between 0.1mg to 1mg, suitably 1ng -50 micrograms. For example, in human applications 5×10⁴ IU/ml units or less may be used.

The interferon alpha subtype IFN-α14, HYBRID 1 and HYBRID 2 is administered by sublingual administration. In embodiments, the interferon alpha subtype can be administered once a day, twice a day, three times a day or four times a day. Suitably, in sublingual administration, a single dose may be provided each day.

The interferon alpha subtype IFN-α14, HYBRID 1 or HYBRID 2 interrupts the viral activity of the coronavirus. In embodiments, the interferon alpha subtype IFN-α14, HYBRID 1 or HYBRID 2 inhibits the cytopathic effect or the plaque formation of the coronavirus.

The subject can be suffering from a condition caused by the coronavirus. In embodiments, the subject can be suffering from a respiratory tract infection or pneumonia, or Acute Respiratory Distress Syndrome (ARDS).

Typically, the subject is a mammal, in particular a human. In embodiments the subject can be a bird. In embodiments the subject can be an animal.

The method includes the step of administering a therapeutically useful amount of a suitable anti-viral compound. In embodiments, the anti-viral compound is ribavirin. In embodiments in combination with the interferon alpha subtype IFN-α14, HYBRID 1 or HYBRID 2 a further treatment may be selected from Remdesivir, LAM-002A (apilimod - a selective PIKfyve kinase inhibitor), dexamethasone, and Avigan (favilavir).

According to the present invention, there is provided an interferon alpha subtype, wherein the interferon alpha subtype is HYBRID 2 or a functionally active fragment of SEQ ID NO: 3 for use in the treatment and/or prophylaxis of a coronavirus infection and wherein HYBRID 2 comprises or consists of an amino acid sequence SEQ ID NO:3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

In embodiments, the coronavirus is Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19).

In embodiments, the coronavirus is Severe Acute Respiratory Syndrome Coronavirus-1 (SARS-CoV-1).

In embodiments, the coronavirus is Severe Acute Respiratory Syndrome Coronavirus-2 (SARS-CoV-2 or Covid19).

The interferon alpha subtype IFN-α14 comprises or consists of an amino acid sequence SEQ ID NO:1 or a functionally active fragment or variant thereof.

The interferon alpha subtype HYBRID 1 comprises or consists of an amino acid sequence SEQ ID NO:2 or a functionally active fragment or variant thereof.

Also disclosed is an interferon alpha subtype, HYBRID 2 for use in the treatment and/or prophylaxis of a viral infection.

The interferon alpha subtype HYBRID 2 comprises or consists of an amino acid sequence SEQ ID NO:3 or a functionally active fragment thereof.

The therapeutically effective amount of HYBRID 2 is a low dose. In embodiments, the therapeutically effective amount of the interferon alpha subtype HYBRID 2 is between 10⁴ to 5×10⁶ IU units/ml. In embodiments, the therapeutically effective amount of HYBRID 2 is lower than current systemic treatments for coronavirus infection.

The HYBRID 2 is administered in a dose of 5IU/ml, 10IU/ml, 50IU/ml, 1×10²1U/ml, 1×10³IU/ml, 1×10⁴IU/ml, 1×10⁵IU/ml, 1×10⁶IU/ml or 1×10⁷IU/ml.

The HYBRID 2 is administered in a dose of between 0.1mg to 1mg, suitably 1ng -50 micrograms. For example, in human applications 5×10⁴ IU/ml units or less may be used. In animals, e.g. dog, sublingual use may be 10⁴ IU/Kg, for example in 1ml PBS.

In certain embodiments of the disclosure the interferon alpha subtype may be administered sublingually. In embodiments, the method of administration is oral administration. In embodiments, the method of administration is injection.

In embodiments, the therapeutically effective amount of the interferon alpha subtype IFN-α14, HYBRID 1 and HYBRID 2 is a low dose. In embodiments, the therapeutically effective amount of the interferon alpha subtype HYBRID 2 is between 10⁴ to 5×10⁶ IU units/ml. In embodiments, the therapeutically effective amount of the interferon alpha subtype HYBRID 2 is lower than current systemic treatments for coronavirus infection.

The interferon alpha subtype IFN-α14, HYBRID 1 and HYBRID 2 is administered in a dose of 5IU/ml, 10IU/ml, 50IU/ml, 1×10²1U/ml, 1×10³IU/ml, 1×10⁴IU/ml, 1×10⁵IU/ml, 1×10⁶IU/ml or 1×10⁷IU/ml.

The interferon alpha subtype IFN-α14, HYBRID 1 and HYBRID 2 is administered in a dose of between 0.1mg to 1mg, suitably 1ng -50 micrograms. For example, in human applications 5×10⁴IU/ml units or less may be used. In animals, e.g. dog, sublingual use may be 10⁴IU/Kg, for example in 1ml PBS.

The the interferon alpha subtype can be administered once a day, twice a day, three times a day or four times a day. Suitably, in sublingual administration, a single dose may be provided each day.

In embodiments in combination with the interferon alpha subtype HYBRID 2 a further treatment may be selected from Remdesivir, LAM-002A (apilimod - a selective PIKfyve kinase inhibitor), dexamethasone, and Avigan (favilavir).

Also disclosed is use of an interferon alpha subtype, wherein the interferon-alpha subtype is IFN-α14, HYBRID 1, HYBIRD 2 or a combination of any of IFN-α14, HYBRID 1 and HYBRID 2, for example of IFN-α14 and HYBRID 1 or IFN-α14 and HYBRID 2 or HYBRID 1 and HYBRID 2, in the preparation of a medicament for the treatment and/or prophylaxis of viral infection, in particular coronavirus infection.

According to a further aspect of the present invention, there is provided a composition comprising an interferon alpha subtype, wherein the interferon alpha subtype is HYBRID 2, for use in the treatment and/or prophylaxis of a coronavirus infection wherein HYBRID 2 comprises or consists of an amino acid sequence SEQ ID NO:3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

Also disclosed is a pharmaceutical composition comprising an interferon alpha subtype, wherein the interferon alpha subtype is IFN-α14, HYBRID 1, HYBRID 2 or a combination of any of IFN-α14, HYBRID 1 and HYBRID 2, for example of IFN-α14 and HYBRID 2 or HYBRID 1 and HYBRID 2, for use in the treatment and/or prophylaxis of viral infection, in particular coronavirus infection.

In embodiments the composition may provide the interferon alpha subtype HYBRID 2 in combination with a further treatment, wherein the further treatment may be selected from Remdesivir, LAM-002A (apilimod - a selective PIKfyve kinase inhibitor), dexamethasone, and Avigan (favilavir).

Also disclosedis an interferon alpha subtype, wherein the interferon subtype is IFN-α14, HYBRID 1, HYBRID 2 or a combination of any of IFN-α14, HYBRID 1 and HYBRID 2, for example of IFN-α14 and HYBRID 2 or HYBRID 1 and HYBRID 2, for use in modulating an immune response.

Also disclosed is HYBRID 2 for use in modulating an immune response.

Also disclosed is a combination of the interferon alpha subtype IFN-α14, HYBRID 1 or HYBRID 2 with a further treatment selected from Remdesivir, LAM-002A (apilimod - a selective PIKfyve kinase inhibitor), dexamethasone, and Avigan (favilavir)can be used to modulate the immune response.

According to a further aspect of the present invention there is provided the use of an interferon alpha subtype, wherein the interferon alpha subtype is, HYBRID 2 and an anti-viral compound for the treatment or prevention of infection with a virus, in particular coronavirus, and in particular Severe Acute Respiratory System (SARS) Coronavirus-1 (SARS-CoV) or Severe Acute Respiratory Syndrome Coronavirus-2 (SARS-CoV-2 or Covid19).

According to a further aspect of the present invention there is provided the use of HYBRID 2 and an anti-viral compound for the treatment or prevention of infection with a virus, and in particular Severe Acute Respiratory System (SARS) Coronavirus-1 (SARS-CoV) or Severe Acute Respiratory Syndrome Coronavirus-2 (SARS-CoV-2 or Covid19). Suitably a virus may be selected from:
Middle East respiratory syndrome coronavirus (MERS-CoV)
Severe acute respiratory syndrome coronavirus (SARS-CoV).

In embodiments of the aspects of the invention outlined above, the coronavirus is Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19).

In embodiments of the aspects of the invention outlined above, the coronavirus is Severe Acute Respiratory Syndrome Coronavirus-1 (SARS-CoV-1).

In embodiments of the aspects of the invention outlined above, the coronavirus is Severe Acute Respiratory Syndrome Coronavirus-2 (SARS-CoV-2 or Covid19).

Also disclosed is where the interferon alpha subtype IFN-α14 comprises or consists of an amino acid sequence SEQ ID NO:1 or a functionally active fragment or variant thereof.

Also disclosed is wherethe interferon alpha subtype HYBRID 1 comprises or consists of an amino acid sequence SEQ ID NO:2 or a functionally active fragment or variant thereof.

In embodiments of the aspects of the invention outlined above, the interferon alpha subtype HYBRID 2 comprises or consists of an amino acid sequence SEQ ID NO:3 or a functionally active fragment thereof.

In embodiments of the aspects of the invention outlined above, (not claimed) the composition is administered sublingually. This may be particularly advantageous for veterinary treatments. In embodiments, the method of administration is oral administration. In embodiments, the method of administration is injection.

In embodiments of the aspects of the invention outlined above, the therapeutically effective amount of the interferon alpha subtype HYBRID 2 is a low dose. In embodiments, the therapeutically effective amount of the interferon alpha subtype HYBRID 2 is between 10⁴ to 5×10⁶ IU units/ml. In embodiments, the therapeutically effective amount of the interferon alpha subtype HYBRID 2 is lower than current systemic treatments for coronavirus infection. Also disclosed is where the interferon alpha subtype HYBRID 2 is administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml or 1×10⁷IU/ml.

Also disclosed is where the interferon alpha subtype HYBRID 2 is administered in a dose of between 0.1mg to 1mg, suitably 1ng to 50 micrograms. For example, in human applications 5×10⁴ IU/ml units or less may be used.

Also disclosed is where the interferon alpha subtype is administered once a day, twice a day, three times a day or four times a day. Suitably, in sublingual administration, a single dose may be provided each day.

Also disclosed is where the IFN-α subtype comprises, consists of or is IFN-α14 such as a fusion protein, or recombinant protein or the like and in particular which comprises or consists of the amino acid sequence SEQ ID NO:1 or a variant or fragment thereof. In embodiments the IFN-α14 or a HYBRID 1 or HYBRID 2 can be glycosylated.

Also disclosed is where the IFN-α subtype comprises, consists of or is HYBRID 1 such as a fusion protein, or recombinant protein or the like and in particular which comprises or consists of the amino acid sequence SEQ ID NO:2 or a variant or fragment thereof.

Also disclosed is where the IFN-α subtype comprises, consists of or is HYBRID 2 such as a fusion protein, or recombinant protein or the like and in particular which comprises or consists of the amino acid sequence SEQ ID NO:3 or a fragment thereof

Also disclosed is a recombinant polypeptide comprising or consisting of SEQ ID NO:1 or a fragment or variant thereof. The invention extends to nucleic acid sequences derived from the amino acid sequence SEQ ID NO:1.

Also disclosed is a recombinant polypeptide comprising or consisting of SEQ ID NO:2 or a fragment or variant thereof. The invention extends to nucleic acid sequences derived from the amino acid sequence SEQ ID NO:2.

Also disclosed is a recombinant polypeptide comprising or consisting of SEQ ID NO:3 or a fragment variant thereof. The invention extends to nucleic acid sequences derived from the amino acid sequence SEQ ID NO:3.

### Description

The inventor of the present invention has surprisingly discovered that administering IFN-α14, for example SEQ ID NO:1 or a variant or fragment thereof, IFN-α14/IFN-α10 hybrids such as HYBRID 1, for example SEQ ID NO:2 or a variant or fragment thereof or HYBRID 2, for example SEQ ID NO:3 or a fragment thereof, as described herein results in the suppression or inhibition of viral activities associated with the immune response in coronavirus infection. Suitably administering IFN-α14, for example SEQ ID NO:1 or a variant or fragment thereof, IFN-α14/IFN-α10 hybrids such as HYBRID 1, for example SEQ ID NO:2 or a variant or fragment thereof or HYBRID 2, for example SEQ ID NO:3 or a fragment thereof, as described herein can be used to treat coronavirus infection in particular severe Acute Respiratory Syndrome Coronavirus-2 (SARS-CoV-2 or Covid19. Surprisingly, this effect may be enhanced when the IFN-α14, HYBRID 1 or HYBRID 2 are administered sublingually.
SEQ ID NO:1 is IFNα-14 and can be defined as follows: 
CNLSQTHSLNNRRTLMLMA QMRRISPFSCLKDRHDFEFP
QEEFDGNQFQKAQAISVLHE MMQQTFNLFSTKNSSAAWDE
TLLEKFYIELFQQMNDLEAC VIQEVGVEETPLMNEDSILA
VKKYFQRITLYLMEKKYSPC AWEVVRAEIMRSLSFSTNLQ KRLRRKD
SEQ ID NO:2 is HYBRID 1 and can be defined as follows: 
CDLPQTHSLGNRRALILLGQMGRISPFSCLKDRHDFRIPQEEFDGNQFQKAQAISVLHEMMQQTFN
LFSTENSSAAWEQTLLEKFSIELFQQMNDLEACVIQEVGVEETPLMNEDSILAVRKYFQRITLYLIE
RKYSPCAWEVVRAEIMRSLSFSTNLQKRLRRKD
SEQ ID NO:3 is HYBRID 2 and can be defined as follows:

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD

In particular, the inventor has discovered that IFN-α14, in particular SEQ ID NO:1, HYBRID 1 (SEQ ID NO:2) or HYBRID 2 (SEQ ID NO:3) or a variant or fragment thereof, inhibits the cytopathic effect and plaque formation as a result of coronavirus infection. The cytopathic effect or cytopathogenic effect refers to structural changes in host cells that are caused by viral invasion.

In particular, the inventor has discovered that HYBRID 2 (SEQ ID NO:3) or a variant or fragment thereof, inhibits the cytopathic effect and plaque formation. The cytopathic effect or cytopathogenic effect refers to structural changes in host cells that are caused by viral invasion.

The inventor has also established that the recombinant IFN-hybrid molecule HYBRID 2 (SEQ ID NO: 3) has a high binding affinity to the interferon receptors.

The inventors have demonstrated that the natural molecule IFNα-14, in particular SEQ ID NO:1, SEQ ID NO:2, SEQ ID NO;3 or a variant or fragment thereof, blocks or inhibits the action of the coronavirus at very low doses. These findings can be applied to provide an improved method and improved composition for treating and/or preventing a coronavirus infection, in particular from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19).

Interferon subtypes IFN-α10 and IFN-α14 and hybrids thereof are discussed in PCT Publication Number WO2014/037717 and PCT Publication Number WO2015/136287. In particular, IFN-α10-IFN-α14 hybrids are disclosed that contain sequences characteristic of the IFN-α10 and IFN-α14 subtype binding sites based on a consensus backbone sequence of all 12 alpha-interferons. Interferon subtypes IFN-α10 and IFN-α14 and hybrids thereof are also discussed in PCT Publication Number WO2017/046583. In particular, IFN-α10-IFN-α14 hybrids are disclosed that have high affinity binding sites derived from IFN-α10 and IFN-α14 subtypes that are not based on a consensus sequence of all 12 IFN-α subtypes. The hybrids derive the sequence characteristics of IFN-α10 and IFN-α14 subtypes without the sequence characteristics of the other 10 interferon-alpha subtypes.

The inventor has also established that the recombinant IFN-hybrid molecules known herein as HYBRID 1 (SEQ ID: 2) and HYBRID 2 (SEQ ID NO: 3) also have a high binding affinity to the interferon receptors.

Whilst not wishing to be bound by theory, the inventor believes that proteins comprising the amino acid sequence of IFN-α10 have greater affinity to interferon receptor 2 (IFNR2) and proteins comprising the amino acid sequence of IFN-α14 have greater affinity to interferon receptor 1 (IFNR1). Thus, substitution of a protein comprising an IFN-α10 amino acid sequence with amino acids of IFN-α14 which allow binding to interferon receptor 1 or substitution of a protein comprising an IFN-α14 amino acid sequence with amino acids of IFN-α10 which allow binding to interferon receptor 2 is considered to provide a IFN-α10 IFN-α14 hybrid protein which should have stronger binding affinity to both interferon receptors 1 and 2 than IFN-α10 or IFN-α14 alone. By including the primary interferon receptor binding sites of IFN-α10 and IFN-α14 is meant that the hybrid comprises amino acids selected from IFN-α10 and substituted into an IFN-α14 amino acid sequence to improve the ability of an IFN-α14 subtype to bind to an interferon receptor 2 and / or that the hybrid comprises amino acids selected from IFN-α14 and substituted into an IFN-α10 amino acid sequence to improve the ability of an IFN-α10 subtype to bind to an interferon receptor 1.

Suitably, several amino acid substitutions of protein comprising an IFN-α10 amino acid sequence with amino acids of IFN-α14 determined to be involved in binding to interferon receptor 1 may enhance the binding of the protein to interferon receptor 1. Suitably, an amino acid substitution of protein comprising an IFN-α14 amino acid sequence with amino acids of IFN-α10 determined to be involved in binding to interferon receptor 2 may enhance the binding of the protein to interferon receptor 2.

In embodiments the IFN-α10-IFN-α14 hybrid can substantially have the amino-acid sequence of IFN-α10, but be modified in a region between amino residues 80 to 150, or suitably between amino acid residues 84 to 144, or suitably amino acid residues 92 to 115 or suitably between amino acid residues 90 to 110, (utilizing the numbering of the IFN-α10 sequence) to provide the amino acids provided by the IFN-α14 sequence. It is considered the amino acid residues in these regions or parts of these regions provide for the binding of IFN-α14 to interferon receptor 1. In particular, the hybrid sequence may include at least one, at least two, at least three, at least 4, at least 5, at least 6, at least 7, at least 8, at least 9, at least 10 or at least 11 modifications of the IFN-α10 sequence to provide the corresponding residues of the IFN-α14 sequence or a conserved mutation thereof. In embodiments, eleven modifications are provided as indicated by the amino acids noted in bold.

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD
(HYBRID 2: SEQ ID NO: 3).

In embodiments, the IFN-α10-IFN-α14 hybrid sequence may include at least one mutation selected from amino acids at positions 94, 101, 102, 109 or 144, preferably at least two mutations selected from amino acids at positions 94, 101, 102, 109 or 144, more preferably at least three mutations selected from amino acids at positions 94,101,102,109 or 144, more preferably at least four mutations selected from amino acids at positions 94, 101, 102, 109 or 144 or more preferably at least five mutations selected from amino acids at positions 94, 101, 102,109 or 144. In alternative embodiments, IFN-α14 can be utilised as a backbone structure of the hybrid and the residues which differ between the IFN-α10 and IFN-α14 sequences at the N and C terminal regions of the sequences can be provided in the hybrid sequence as those present in the IFN-α10 sequence. Suitably at least 1, at least 2, at least 3, at least 4, at least 5, at least 6, at least 7, at least 8, at least 9, at least 10 or at least 11 substitutions of the IFN-α14 N-terminal sequence may be made to provide the hybrid sequence to provide residues from IFN-α10 at those amino acid positions wherein the amino acids are not shared/common between IFN-α10 and IFN-α14. Suitably, at least 1, at least 2, or 3 substitutions are provided at the IFN-α14 C terminal sequence to provide residues from IFN-α10 to the hybrid sequence at those amino acid positions which are not shared/common between IFN-α10 and IFN-α14. In embodiments at least 1, at least 2, at least 3, at least 4, at least 5, at least 6, at least 7, at least 8, at least 9, at least 10 or at least 11 substitutions from the N-terminal sequence and at least 1, at least 2, or 3 substitutions from the C-terminal sequence of the IFN-α14 are made to provide residues from IFN-α10 to the hybrid at those amino acid positions which have amino acids that are not shared/common between IFN-α10 and IFN-α14.

By functionally active is meant an IFN-α10-IFN- α14 hybrid polypeptide comprising the primary interferon binding sites of IFN-α10 and IFN-α14 wherein the administration of peptide to a subject or expression of peptide in a subject promotes suppression of an immune response to a coronavirus infection. Further, functional activity may be indicated by the ability of a hybrid peptide to suppress an immune response to a coronavirus infection.

A fragment can comprise at least 50, preferably 100 and more preferably 150 or greater contiguous amino acids from SEQ ID NO: 1, 2 or 3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein.

By variant is meant an amino acid sequence which is at least 70% homologous to SEQ ID NO: 1, 2 or 3, more preferably at least 80% homologous to SEQ ID NO: 1, 2 or 3, more preferably at least 90% homologous to SEQ ID NO: 1, 2 or 3, even more preferably at least 95% homologous to SEQ ID NO: 1, 2 or 3, even more preferably at least 96% homologous to SEQ ID NO: 1, 2 or 3, even more preferably at least 97% homologous to SEQ ID NO: 1, 2 or 3 and most preferably at least 98% homology with SEQ ID NO: 1, 2 or 3, at least 99% homologous to SEQ ID NO:1, 2 or 3. A variant encompasses a polypeptide sequence of SEQ ID NO: 1, 2 or 3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Hybrids, and variants and fragments thereof may be generated using suitable molecular biology methods as known in the art. Suitably homology may be described by sequence identity. Sequence identity can be determined by methods as known in the art, for example BLAST.

There are many differences between the recombinant forms of IFN alpha in the art and the multi-subtype forms. The most obvious difference is the number of IFN alpha subtypes each possesses. The recombinant forms comprise only the alpha 2 subtype - the alpha 2b form for Intron A^{™} (Schering Plough) and the alpha 2a form for Roferon^{™} (Roche). The multi-subtype forms of IFN alpha comprise many subtypes of IFN alpha. The multi-subtype forms of IFN alpha are a broad spectrum drug and there is no indication that any one of the IFN alpha subtypes has more or less anti-viral activity. Another difference between the multi-subtype and the recombinant forms is that the IFN alpha 2 produced by human cells in the manufacturing process of the multi-subtype forms is glycosylated, whereas the recombinant forms are unglycosylated, in that they are produced through bacterial fermentation. Glycosylation plays a major role in many functions of the protein product, such as half-life, the bioactivity and its immunogenicity. Therefore, the glycosylation of a product is an important consideration when developing a therapeutic or prophylactic treatment, as it may affect the duration in the body after administration, the activity of a therapeutically appropriate dose and the tolerability to the product itself.

The inventor has discovered that administration of IFN-α14, in particular SEQ ID NO:1, HYBRID 1 (SEQ ID NO:2) or HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation, compared to controls to which the IFN-α14, in particular SEQ ID NO:1, HYBRID 1 (SEQ ID NO:2) or HYBRID 2 (SEQ ID NO:3) or a variant or fragment thereof have not been administered.

The inventor has discovered that administration of IFN-α14, in particular SEQ ID NO:1, HYBRID 1 (SEQ ID NO:2) or HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation, compared to previous anti-viral medications.

The inventor has discovered that administration of HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation, compared to controls to which HYBRID 2 (SEQ ID NO:3) or a variant or fragment thereof have not been administered.

The inventor has discovered that administration of HYBRID 2 (SEQ ID NO:3) or a variant or fragment thereof, results in a 10%, preferably a 20%, preferably a 30%, preferably a 40%, preferably a 50%, preferably a 60%, preferably a 70%, preferably a 80% and more preferably a 90% greater reduction of viral activity, such as cytopathic effect or plaque formation, compared to previous anti-viral medications.

Administration of HYBRID 2 can reduce cytopathic effect or plaque formation by 50%, preferably by 60%, preferably by 70%, preferably by 80%, preferably by 90%, preferably by 91%, preferably by 92%, preferably by 93%, preferably by 94%, preferably by 95%, preferably by 96%, preferably by 97%, and more preferably by 98%.

The inventor has surprisingly discovered that administration of IFN-α14, in particular SEQ ID NO:1 or a variant or fragment thereof, results in the inhibition of the cytopathic effect or plaque formation as a result of coronavirus infection by 50%, preferably by 60%, preferably by 70%, preferably by 80%, preferably by 90%, preferably by 91%, preferably by 92%, preferably by 93%, preferably by 94%, preferably by 95%, preferably by 96%, preferably by 97%, and more preferably by 98%. HYBRID 1 and HYBRID 2 show similar functional effects. HYBRID 2 is considered to be particularly effective at reducing viral activity.

The inventor has surprisingly discovered that administration of IFN-α14, in particular SEQ ID NO:1 or a variant or fragment thereof, results in the suppression of the cytopathic effect or plaque formation at low doses as discussed herein. HYBRID 1 and HYBRID 2 show similar functional effects. HYBRID 2 is considered to be particularly effective at reducing viral activity.

Treatment of the present invention can be administered in a dose of 5IU/ml, 10IU/ml, 50IU/ml, 1×10²IU/ml, 1×10³IU/ml, 1×10⁴IU/ml, 1×10⁵IU/ml, 1×10⁶IU/ml, 1×10⁷IU/ml or 1×10⁸IU/ml.

The treatment of the present invention can be administered in a dose of suitably 1ng -50 micrograms, 0.1mg to 1mg, 1mg to 3mg, 3mg to 5mg or 5mg to 10mg.

The treatment of the present invention can be sublingually administered once a day, twice a day, three times a day or at least four times a day. The treatment of the present invention can be administered by aerosol delivery into the lungs once a day, twice a day, three times a day or at least four times a day. The treatment of the present invention can be administered by injection, once a day, twice a day, three times a day or at least four times a day.

In addition, the inventor has discovered that the administration or use of IFN-α14, in particular SEQ ID NO:1, HYBRID 1 (SEQ ID NO:2) or HYBRID 2 (SEQ ID NO:3) or a variant or fragment thereof, results in the full or partial inhibition of coronaviral activity and/or the full or partial inhibition of the cytopathic effect as a result of coronavirus infection and/or the full or partial inhibition of plaque formation as a result of coronavirus infection.

The present invention provides a superior treatment that is safe and effective from coronavirus infection, in particular from Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19). This treatment demonstrates a low side-effect profile. Low doses of the medication are required and the natural product, IFN-α14, HYBRID 1, or HYBRID 2 displays no cytotoxicity in-vitro at even the highest concentration (1×10⁸ IU/ml).

The inventor has demonstrated that the addition of Interferon-alpha-14, in particular SEQ ID NO:1, HYBRID 1, in particular SEQ ID NO:2 or HYBRID 2, in particular SEQ ID NO:3, or a variant or fragment thereof, resulted in strong inhibition of coronavirus activity. The present inventor has demonstrated the ability of the synthetic alpha-interferon, Interferon-alpha-14 (SEQ ID NO:1) to inhibit the cytopathic effect and plaque formation caused as a result of infection with SARS-CoV-1. The inventor has examined the inhibition of the cytopathic effect in comparison with the anti-viral compound Ribavirin and the multi-subtype interferon Multiferon^{™}. Surprisingly Interferon-alpha14 (SEQ ID NO:1) is active against SARS-CoV-1 and SARS-CoV- 2 while Ribavirin and the multi-subtype interferon Multiferon^{™} show much less inhibitory activity. HYBRID 1 and HYBRID 2 show similar functional effects. These results are a clear indication of the potential superiority of IFNα-14, in particular SEQ ID NO:1, HYBRID 1, in particular SEQ ID NO:2 or HYBRID 2, in particular SEQ ID NO:3 or a variant or fragment thereof, in the treatment of a coronavirus infection over existing anti-viral treatments.

### Definitions

### Fragment

A fragment can comprise at least 50, preferably 100 and more preferably 150 or greater contiguous amino acids from SEQ ID NO: 1, SEQ ID NO:2 or SEQ ID NO:3 and which is functionally active. Suitably, a fragment may be determined using, for example, C-terminal serial deletion of cDNA. Said deletion constructs may then be cloned into suitable plasmids. The activity of these deletion mutants may then be tested for biological activity as described herein. Fragments may be generated using suitable molecular biology methods as known in the art.

### Variant

By variant is meant an amino acid sequence which is at least 70% homologous to SEQ ID NO: 1, SEQ ID NO:2 or SEQ ID NO:3, more preferably at least 80% homologous to SEQ ID NO: 1, SEQ ID NO:2 or SEQ ID NO:3, more preferably at least 90% homologous to SEQ ID NO: 1, SEQ ID NO:2 or SEQ ID NO:3, even more preferably at least 95% homologous to SEQ ID NO: 1, SEQ ID NO:2 or SEQ ID NO:3, even more preferably at least 96% homologous to SEQ ID NO: 1, SEQ ID NO:2 or SEQ ID NO:3, even more preferably at least 97% homologous to SEQ ID NO: 1, SEQ ID NO:2 or SEQ ID NO:3, and most preferably at least 98% homology with SEQ ID NO: 1, SEQ ID NO:2 or SEQ ID NO:3. A variant encompasses a polypeptide sequence of SEQ ID NO: 1, SEQ ID NO:2 or SEQ ID NO:3 which includes substitution of amino acids, especially a substitution(s) which is/are known for having a high probability of not leading to any significant modification of the biological activity or configuration, or folding, of the protein. These substitutions, typically known as conserved substitutions, are known in the art. For example the group of arginine, lysine and histidine are known interchangeable basic amino acids. Suitably, in embodiments amino acids of the same charge, size or hydrophobicity may be substituted with each other. Suitably, any substitution may be selected based on analysis of amino acid sequence alignments of interferon alpha subtypes to provide amino acid substitutions to amino acids which are present in other alpha subtypes at similar or identical positions when the sequences are aligned. Variants may be generated using suitable molecular biology methods as known in the art.

### Subject

As herein defined, a "subject" includes and encompasses mammals such as humans, primates and livestock animals (e.g. sheep, pigs, cattle, horses, donkeys); laboratory test animals such as mice, rabbits, rats and guinea pigs; and companion animals such as dogs and cats.

### Treatment / Therapy

The term "treatment" is used herein to refer to any regimen that can benefit a human or nonhuman animal. The treatment may be in respect of a coronavirus infection and the treatment may be prophylactic (preventative treatment). Treatment may include curative or alleviative effects. Reference herein to "therapeutic" and "prophylactic" treatment is to be considered in its broadest context. The term "therapeutic" does not necessarily imply that a subject is treated until total recovery. Similarly, "prophylactic" does not necessarily mean that the subject will not eventually contract a disease condition. Accordingly, therapeutic and/or prophylactic treatment includes amelioration of the symptoms of a coronavirus infection or preventing or otherwise reducing the risk of developing a coronavirus infection. The term "prophylactic" may be considered as reducing the severity or the onset of a particular condition. "Therapeutic" may also reduce the severity of an existing condition.

### Administration

The active ingredients used in the present invention in particular the interferon subtype IFN-α14, for example SEQ ID NO: 1, HYBRID 1 (SEQ ID NO:2) or HYBRID 2 (SEQ ID NO:3), as described herein can be administered separately to the same subject, optionally sequentially, or can be co-administered simultaneously as a pharmaceutical or immunogenic composition.

Interferons of and for use in the present invention may be administered alone, or in combination with another agent, but will preferably be administered as a pharmaceutical composition. The pharmaceutical composition will generally comprise a suitable pharmaceutical excipient, diluent or carrier selected depending on the intended route of administration.

The active ingredients can be administered to a patient in need of treatment via any suitable route. The precise dose will depend upon a number of factors, as is discussed below in more detail.

Some suitable routes of administration include (but are not limited to) oral, rectal, nasal, topical (including buccal and sublingual), vaginal or parenteral (including subcutaneous, intramuscular, intravenous, intradermal, intrathecal and epidural) administration, or administration via oral or nasal inhalation.

The composition is deliverable as an injectable composition, is administered orally, is administered to the lungs as an aerosol via oral or nasal inhalation.

One suitable route of administration is sublingually, e.g. applied under the subject's tongue.

For administration via the oral or nasal inhalation routes, preferably the active ingredient will be in a suitable pharmaceutical formulation and may be delivered using a mechanical form including, but not restricted to an inhaler or nebuliser device.

Further, where the oral or nasal inhalation routes are used, administration by a SPAG (small particulate aerosol generator) may be used.

For intravenous injection, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

The composition may also be administered via microspheres, liposomes, other microparticulate delivery systems or sustained release formulations placed in certain tissues including blood. Suitable examples of sustained release carriers include semipermeable polymer matrices in the form of shared articles, e.g. suppositories or microcapsules. Implantable or microcapsular sustained release matrices include polylactides copolymers of L-glutamic acid and gamma ethyl-L-glutamate, poly (2-hydroxyethyl-methacrylate) or ethylene vinyl acetate.

Examples of the techniques and protocols mentioned above and other techniques and protocols which may be used in accordance with the invention can be found in Remington's Pharmaceutical Sciences, 16th edition, Oslo, A. (ed), 1980.

### Pharmaceutical Compositions

As described above, the present invention extends to a pharmaceutical composition for the treatment of a coronavirus infection.

Pharmaceutical compositions according to the present invention, and for use in accordance with the present invention, may comprise, in addition to an active ingredient, a pharmaceutically acceptable excipient, carrier, buffer stabiliser or other materials well known to those skilled in the art. Such materials should be non-toxic and should not interfere with the efficacy of the active ingredient. The precise nature of the carrier or other material will depend on the route of administration, which may be, for example, oral, intravenous, intranasal or via oral or nasal inhalation. The formulation may be a liquid, for example, a physiologic salt solution containing non-phosphate buffer at pH 6.8-7.6, or a lyophilised or freeze-dried powder.

### Dose

The composition is preferably administered to an individual in a "therapeutically effective amount" or a "desired amount", this being sufficient to show benefit to the individual. As defined herein, the term an "effective amount" means an amount necessary to at least partly obtain the desired response, or to delay the onset or inhibit progression or halt altogether the onset or progression of a particular condition being treated. The amount varies depending upon the health and physical condition of the subject being treated, the taxonomic group of the subject being treated, the degree of protection desired, the formulation of the composition, the assessment of the medical situation and other relevant factors. It is expected that the amount will fall in a relatively broad range, which may be determined through routine trials. Prescription of treatment, e.g. decisions on dosage etc., is ultimately within the responsibility and at the discretion of general practitioners, physicians or other medical doctors, and typically takes account of the disorder to be treated, the condition of the individual patient, the site of delivery, the method of administration and other factors known to practitioners. The optimal dose can be determined by physicians based on a number of parameters including, for example, age, sex, weight, severity of the condition being treated, the active ingredient being administered and the route of administration. A broad range of doses may be applicable. Considering oral administration to a human patient, for example, from about 10 µg to about 1000 µg of agent may be administered per human dose, optionally for 3 to 4 doses. Dosage regimes may be adjusted to provide the optimum therapeutic response and reduce side effects. For example, several divided doses may be administered daily, weekly, monthly or other suitable time intervals or the dose may be proportionally reduced as indicated by the exigencies of the situation.

Unless otherwise defined, all technical and scientific terms used herein have the meaning commonly understood by a person who is skilled in the art in the field of the present invention.

### Brief description of the Figures

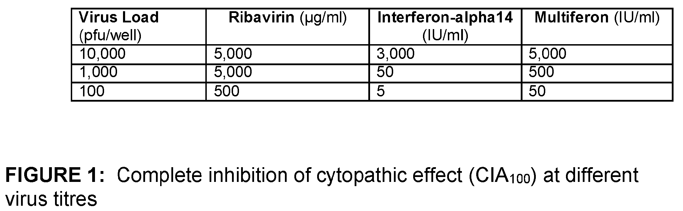

Figure 1 shows the effect of IFNα-14 on the cytopathic effect following infection from SARS-CoV-1 Coronavirus.

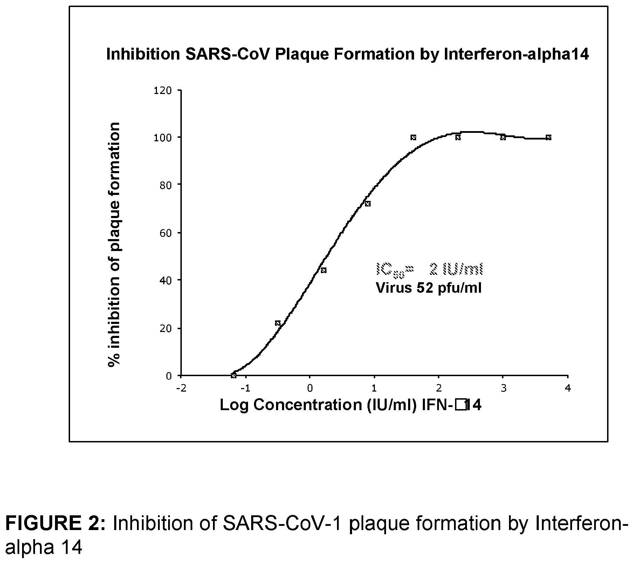

Figure 2 shows a graph demonstrating the effect of IFNα-14 on plaque formation following infection from SARS-CoV-1 Coronavirus.
Figure 3 shows results of Plaque Inhibition Assays using Vero Cells of a SARS-CoV-2 Inhibition plaque assay in which IFNalpha 2a (the commercially available subtype) is compared with IFNalpha 14 and shows the IC50s - this indicates Alpha-14 is 20X more potent against the Covid 19 virus than Alpha-2a
Figure 4 shows results of Plaque Inhibition Assays using Vero Cells of studies comparing 5 interferons - INTERFERON-ALPHA 14, HYBRID 2, HYBRID 1, INTERFERON-BETA 1a, INTERFERON -ALPHA 2a - this gives a comparison based again on Alpha-2a of the 4 other 'INTERFERONS' of 20X, 10X, 5X more potent than Alpha-2a.
Figure 5 shows the effect of INTERFERON-ALPHA 14, on CXCL-10 synthesis by Human Endothelial cells. CXCL-10 is the attractant for anti-viral NK cells.
Figure 6 shows rapid induction of Granzyme B with INTERFERON ALPHA 14. Granzyme B causes release to kill cells with virus.
Figure 7 shows suppression of IL-17A synthesis by Hybrid 2. INTERFERON ALPHA 14 inhibits both 17 A and F very strongly. IL17 inflammation is considered to be a significant issue in ARDS.
Figure 8 shows suppression of IL 17F with Hybrid 2.
Figure 9 shows the effect of Hybrid 2 on the production of CXCL 10 from human leucocytes.
Figure 10 shows interferon gamma production.
Figure 11 shows TNF alpha secretion.
Figure 12 shows images from immune cells and Hybrid 2.
Figure 13 shows Hybrid 2 can induce apoptosis.
Figure 14 shows NK cell clustering.
Figure 15 shows direct activation of NK cells by Hybrid 2.

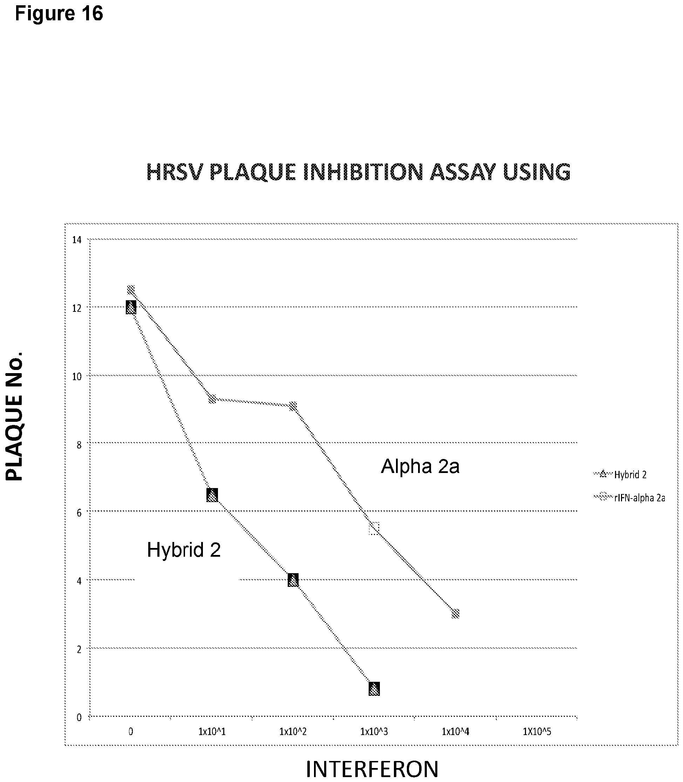

Figure 16 shows HYBRID 2's inhibition of a plaque assay with human RSV.

### Experimental Data

The present inventor has examined the ability of the synthetic alpha-interferon Hybrid 2 to inhibit viral infection using a model system based on the cytopathic effect and plaque formation caused as a result of infection with SARS-CoV-1 and SARS-CoV-2. The inventor has also examined the inhibition of the cytopathic effect in comparison with the anti-viral compound Ribavirin and the multi-subtype interferon Multiferon^{™}. Additionally the inventor has utilised a RSV to demonstrate the anti-viral effect of HYBRID 2.

RSV infection is the most important cause of hospitalisation in infants and one of the leading causes of infant mortality. As one of the respiratory viruses, along with influenza, rhinoviruses and coronaviruses, it is both interesting in its own right and provides an indicative model.

### Experiment 1: The effect of IFNα-14 on the cytopathic effect following infection from SARS-CoV-1 Coronavirus.

The effectiveness of the interferon alpha subtype IFN-α14 to inhibit the cytopathic effect following SARS-CoV-1 infection was tested in a cytopathic endpoint assay. All endpoint assays were carried out using the multi-subtype Multiferon and IFN-α 14 as well as the antiviral Ribavirin for comparison.

### Preparation of anti-viral treatments.

A broad range of concentrations (obtained by ten-fold dilutions) encompassing the inhibitory dosages commonly used for other viral-host combinations was tested. Compounds were dissolved Hank's buffered-saline solution.

For plaque assays, 5-fold drug dilutions were prepared using growth media as specified below. SARS-CoV-1 production and infection African Green Monkey (Vero E6) cells (American Type Culture Collection, Manassas, VA, USA) were propagated in 75cm cell culture flasks containing growth medium consisting of Medium 199 (Sigma, St Louis, USA) supplemented with 10% foetal calf serum (FCS; Biological Industries, Israel). SARS-HCoV2003VA2774 (an isolate from a SARS patient in Singapore) was propagated in Vero E6 cells. Briefly, 2 ml of stock virus was added to a confluent monolayer of Vero E6 cells and incubated at 37°C in 5% C0₂ for one hour. 13 ml Medium 199, supplemented with 5% FCS, was then added. The cultures were incubated at 37°C in 5% C0₂ and the inhibition of the cytopathic effect gauged by observing each well through an inverted microscope. Where 75% or greater inhibition was observed after 48 hours, the supernatant was harvested. The supernatant was clarified at 2500 rpm and then aliquoted into cryovials and stored at -80°C until use.

### Virus handling and titration

Virus titre in the frozen culture supernatant was determined using a plaque assay carried out in duplicate. Briefly, 100 microlitres of virus in a 10-fold serial dilution was added to a monolayer of Vero E6 cells in a 24 well-plate. After incubation for an hour at 37°C in 5% C0₂, the viral Medium 199 supplemented with 5% FCS was added. Cells were fixed with 10%(v/v) formalin and stained with 2% (w/v) crystal violet. The plaques were counted visually and the virus titre in plaque forming units per ml (pfu/ml) calculated.

### Cytopathic endpoint assay

The effect of each anti-viral treatment was tested in quadruplicate. Briefly, 100 microlitres of serial 10-fold dilutions of each treatment were incubated with 100 microlitres of Vero E6 cells giving a final cell count of 20,000 cells per well in a 96-well plate. Incubation was at 37°C in 5% C0₂ overnight for the interferon preparations and for one hour for the of infection (MOI) (virus particles per cell) of 0. 5. The plates were incubated at 37°C in 5% C0₂ for three days and the plates were observed daily for cytopathic effects. The end point was the diluted concentration that inhibited the cytopathic effect in all four set-ups.

To determine cytotoxicity, 100 microlitres of serial 10-fold dilutions of each of the treatments were incubated with 100 microlitres of Vero E6 cells giving a final cell count of 20,000 cells per well in a 96-well plate, without viral challenge. The plates were then incubated at 37°C in 5% C0₂ for three days and toxicity effects were observed for using an inverted microscope.

10 microlitres of virus at a concentration of 10,000 pfu/well were then added to each test well. This equates to a multiplicity of infection (MOI) (virus particles per cell) of 0.5. The plates were incubated at 37°C in 5% CO₂ for three days and the plates were observed daily for cytopathic effects. The end point was the diluted concentration that inhibited the cytopathic effect in all four set-ups (CIA100).

To determine cytotoxicity, 100 microlitres of serial 10-fold dilutions of each treatment were incubated with 100 microlitres of Vero E6 cells giving a final cell count of 20,000 cells per well in a 96-well plate, without viral challenge. The plates were then incubated at 37°C in 5% C0₂ for three days and toxicity effects were observed for using an inverted microscope. Interferons which showed complete inhibition were tested further at the lower viral titres of 10³ and10² pfu/well.

### Experiment 2: The effect of IFNα-14 on plaque formation following infection from SARS-CoV-1 Coronavirus.

The effectiveness of the interferon alpha subtype IFN-α14 to inhibit plaque formation following SARS-CoV-1 infection was tested in a plaque reduction assay.

The plaque assay was performed using 10-fold dilutions of a virus stock, and 0.1 ml aliquots were inoculated onto susceptible cell monolayers. After an incubation period, which allowed virus to attach to cells, the monolayers were covered with a nutrient medium containing a substance, usually agar, that causes the formation of a gel. After plate incubation, the original infected cells released viral progeny. The spread of the new viruses is restricted to neighbouring cells by the gel. Consequently, each infectious particle produced a circular zone of infected cells called a plaque. Eventually the plaque became large enough to be visible to the naked eye. Dyes to stain living cells were used to enhance the contrast between the living cells and the plaques. Only viruses that caused visible damage to cells were assayed in this way i.e. SARS-CoV-1.

These results clearly demonstrate the ability of the synthetic alpha-interferon, Interferon-alpha-14 (SEQ ID NO:1) to inhibit the cytopathic effect and plaque formation caused as a result of infection with SARS-CoV-1. The results demonstrate the inhibition of the cytopathic effect in comparison with the anti-viral compound Ribavirin and the multi-subtype interferon Multiferon^{™}. Surprisingly Interferon-alpha14 (SEQ ID NO:1) is active against SARS-CoV-1 while Ribavirin and the multi-subtype interferon Multiferon^{™} show much less inhibitory activity. HYBRID 1 and HYBRID 2 show similar functional effects. These results are a clear indication of the potential superiority of IFNα-14, in particular SEQ ID NO:1, HYBRID 1, in particular SEQ ID NO:2 or HYBRID 2, in particular SEQ ID NO:3 or a variant or fragment thereof, for the treatment of a coronavirus infection over existing anti-viral treatments.

### Experiment 3: The effect of IFNα-14 on plaque formation following infection from SARS-CoV-2 Coronavirus.

The effectiveness of the interferon alpha subtype IFN-α14 to inhibit plaque formation following SARS-CoV-2 infection was tested in a plaque reduction assay.

The plaque assay was performed using 10-fold dilutions of a virus stock, and 0.1 ml aliquots were inoculated onto susceptible cell monolayers. After an incubation period, which allowed virus to attach to cells, the monolayers were covered with a nutrient medium containing a substance, usually agar, that causes the formation of a gel. After plate incubation, the original infected cells released viral progeny. The spread of the new viruses is restricted to neighbouring cells by the gel. Consequently, each infectious particle produced a circular zone of infected cells called a plaque. Eventually the plaque became large enough to be visible to the naked eye. Dyes to stain living cells were used to enhance the contrast between the living cells and the plaques. Only viruses that caused visible damage to cells were assayed in this way i.e. SARS-CoV-2.

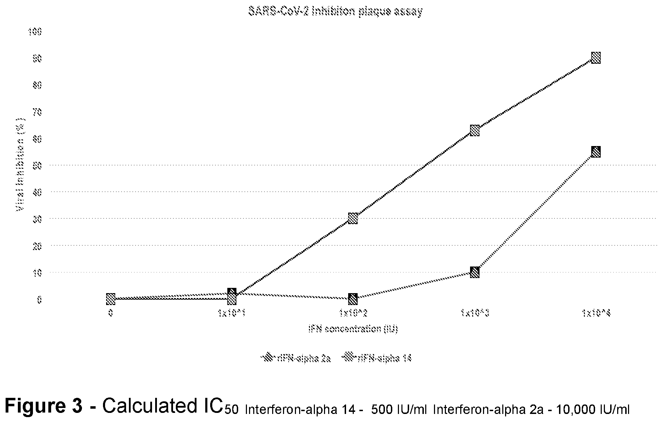

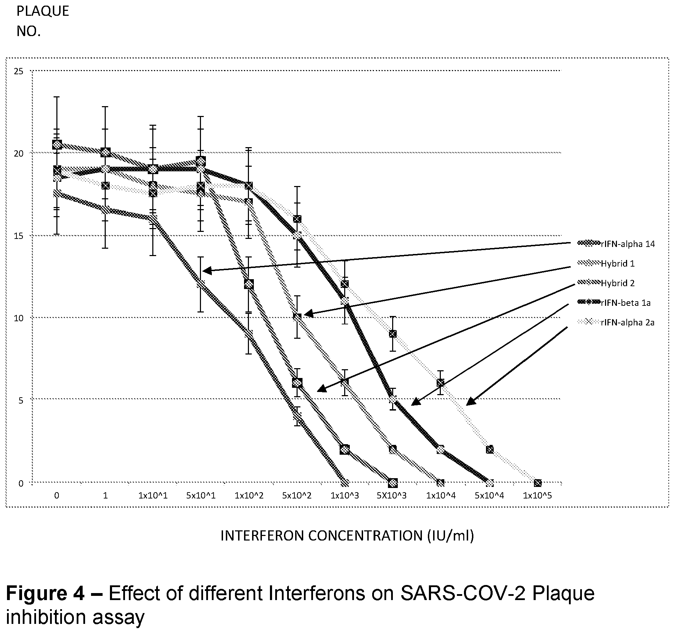

These results (Figures 3 and 4) clearly demonstrate the ability of the synthetic alpha-interferon, Interferon-alpha-14 (SEQ ID NO:1) to inhibit the cytopathic effect and plaque formation caused as a result of infection with SARS-CoV-2. The results demonstrate the inhibition of the cytopathic effect in comparison with the other interferons. Interferon-alpha14 (SEQ ID NO:1), HYBRID 1 and HYBRID 2 are active against SARS-CoV-2. These results are a clear indication of the potential superiority of IFNα-14, in particular SEQ ID NO:1, HYBRID 1, in particular SEQ ID NO:2 or HYBRID 2, in particular SEQ ID NO:3 or a variant or fragment thereof, for the treatment of a coronavirus infection over existing anti-viral treatments.

### Assays in relation IL-17, Granzyme B. Interferon gamma, Interferon alpha, NK cells

Assays utilising interferon alpha 14 and Hybrid 2 in relation to key immune modulators were undertaken as would be understood in the art.

### Effect on HYBRID 2 on immune cell killing

The effect of a hybrid recombinant interferon was tested in a live cell imaging assay of immune cell killing on an IncuCyte ZOOM platform.

SK-OV-3 ovarian cancer cells with red labelled nuclei (SK-OV-3 NucLight Red) were used as target cells in the study. For immune cell killing, the cells were co-cultured with natural killer (NK) cells and positive controls consisted of cells treated with IL-2 and IL-12. Apoptosis was detected by staining caspase 3/7 positive objects while cell number was determined by counting of red nuclei. The results from the co-culture model were compared to those of a mono-culture model consisting of SK-OV-3 NucLight Red cells alone.

An initial optimisation experiment was carried out that tested 4 ratios of target and effector cells. These results determined that 5,000 natural killer cells and 2,000 SK- OV-3 NucLight Red cells per well of a 96-well plate gave a suitable assay window for detecting immune cell killing.

Eight doses of hybrid recombinant interferon ranging from 10 IU/ml to 3×10⁶ IU/ml were tested for effects on immune cell killing. Results were compared to no treatment controls, vehicle treated controls and IL-2/IL-12 treated cells. All conditions were tested using cells in co-culture (SK-OV-3 NucLight and NK cells) and mono-culture (SK-OV-3 NucLight Red).

Cells were monitored for 4 days using an IncuCyte ZOOM, and IncuCyte software was used to measure green (apoptosis) and red (cell number) object count over time. Area under the curve (AUC) analysis was used to quantitate apoptosis and cell number over the time course.

IL-17, Il-6, CCL-5 and the immune response have been determined to be modulated following Covid-19 infection, in particular Covid-19 induced Acute Respiratory Distress Syndrome where IL-17 inflammation has been indicated as being significant.

Hybrid recombinant interferon caused a strong induction in apoptosis in the co-culture model, with an increase in AUC from 200 in vehicle controls to 1174 at the top dose of hybrid recombinant interferon. An EC₅₀ of 1.5x10⁶ IU/ml was derived for apoptosis induction. In contrast, cells in mono-culture displayed only a marginal response to hybrid rIFN.

Hybrid recombinant interferon also caused a reduction in cell number. AUC values for cell number fell from 39921 in vehicle controls to 19501 at the top dose of hybrid rIFN. An IC₅₀ value of 1.3×10⁶ IU/ml was determined for the reduction in cell number.

There was evidence of very strong direct activation of natural killer cells by hybrid rIFN, with cell clustering of NK cells observed in response to hybrid recombinant interferon treatment in a NK cell monoculture model.

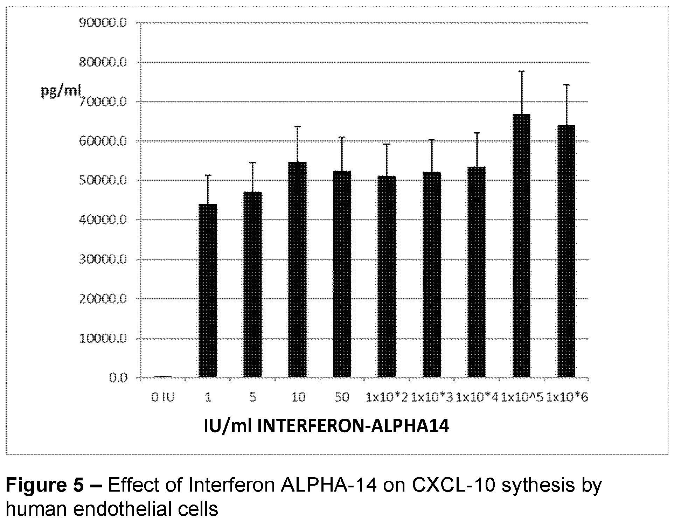

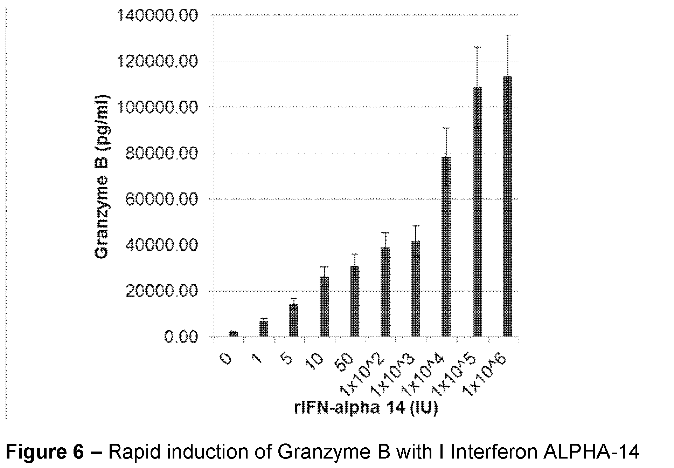

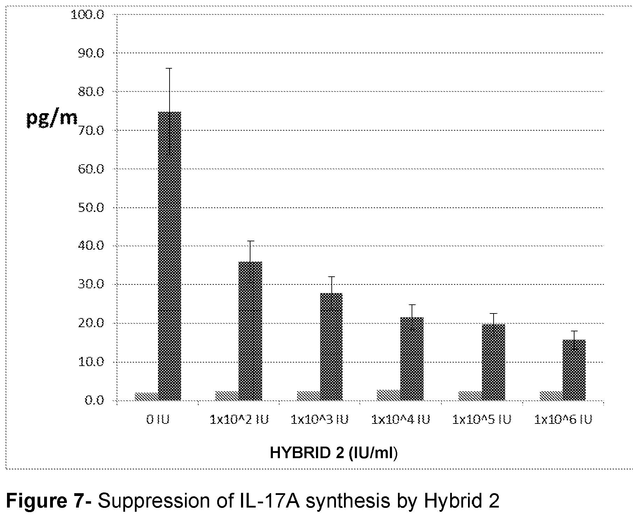

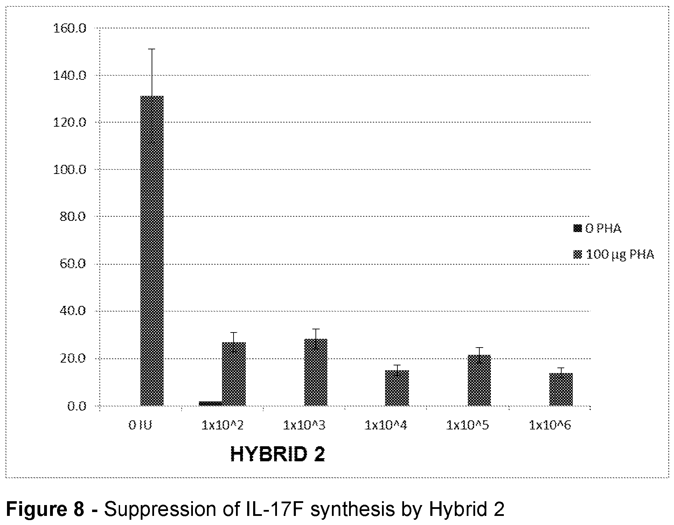

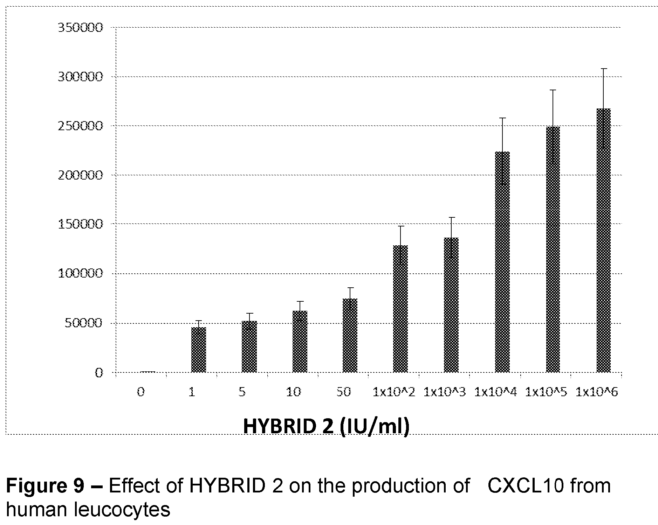

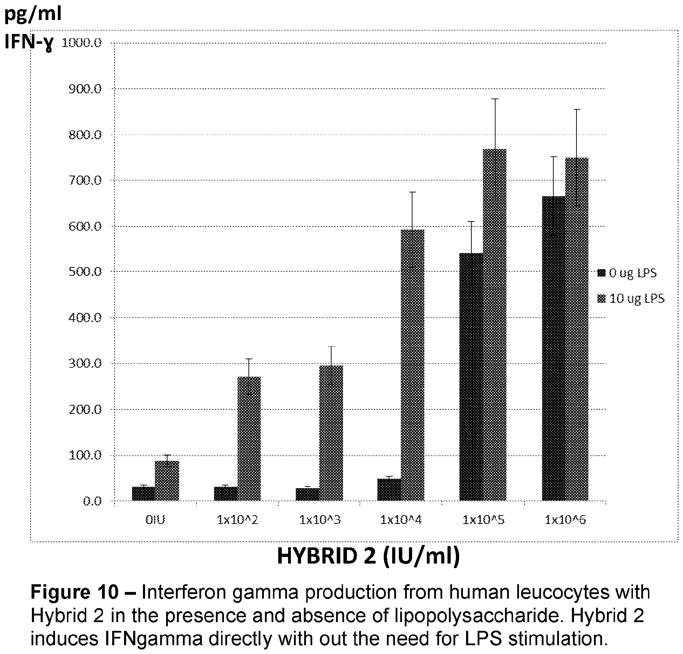

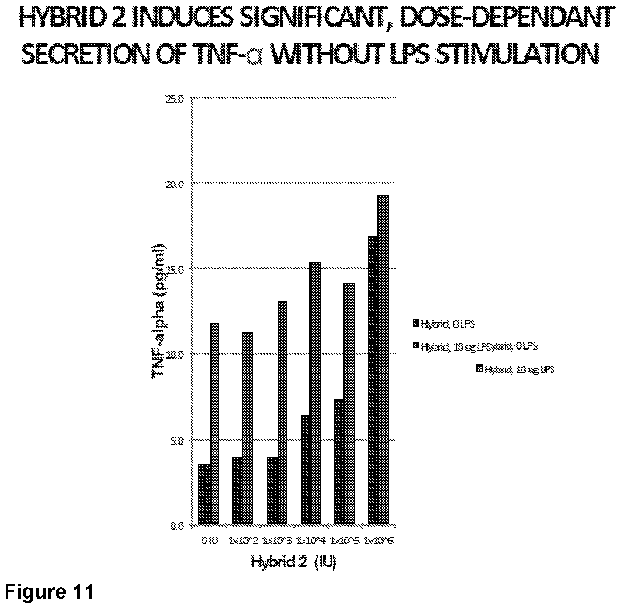

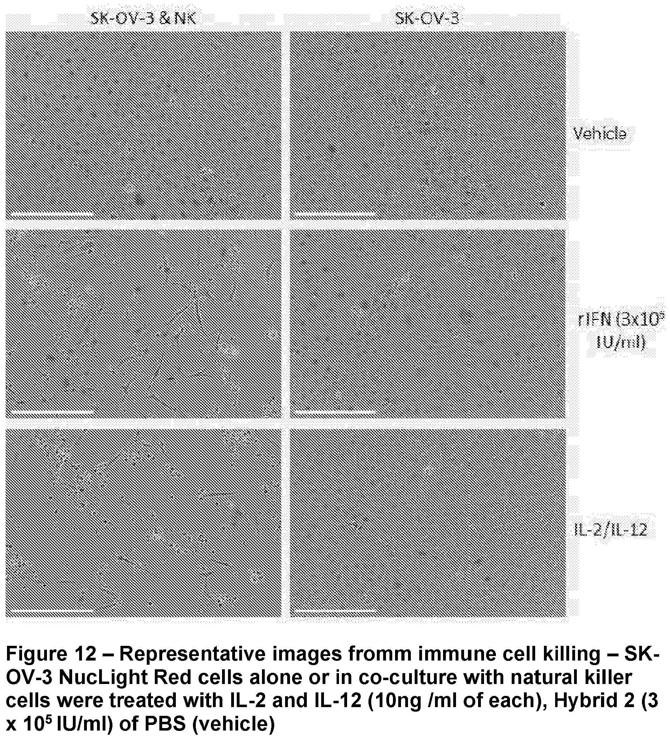

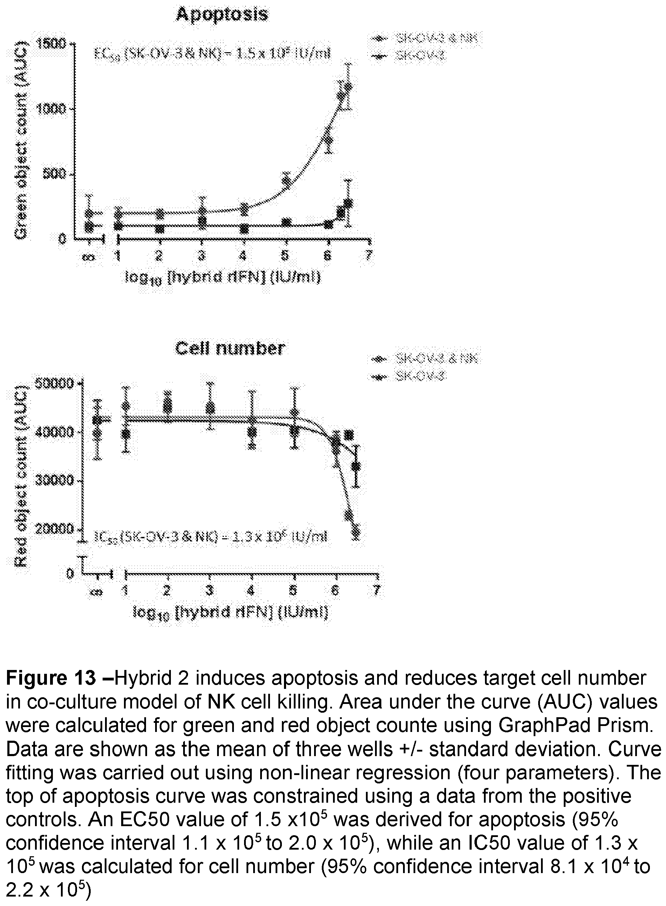

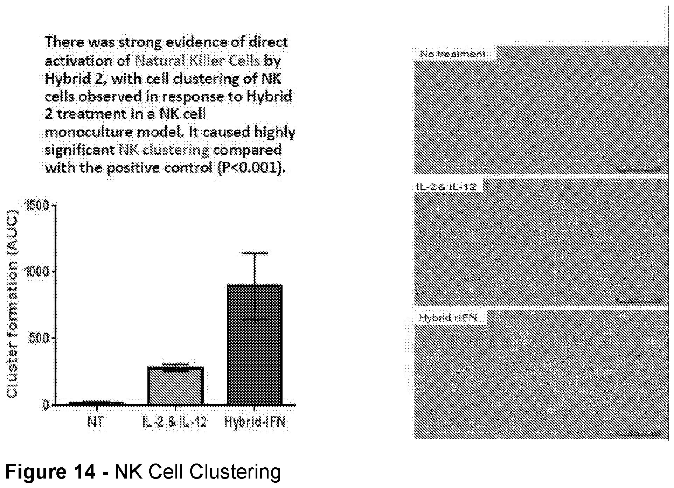

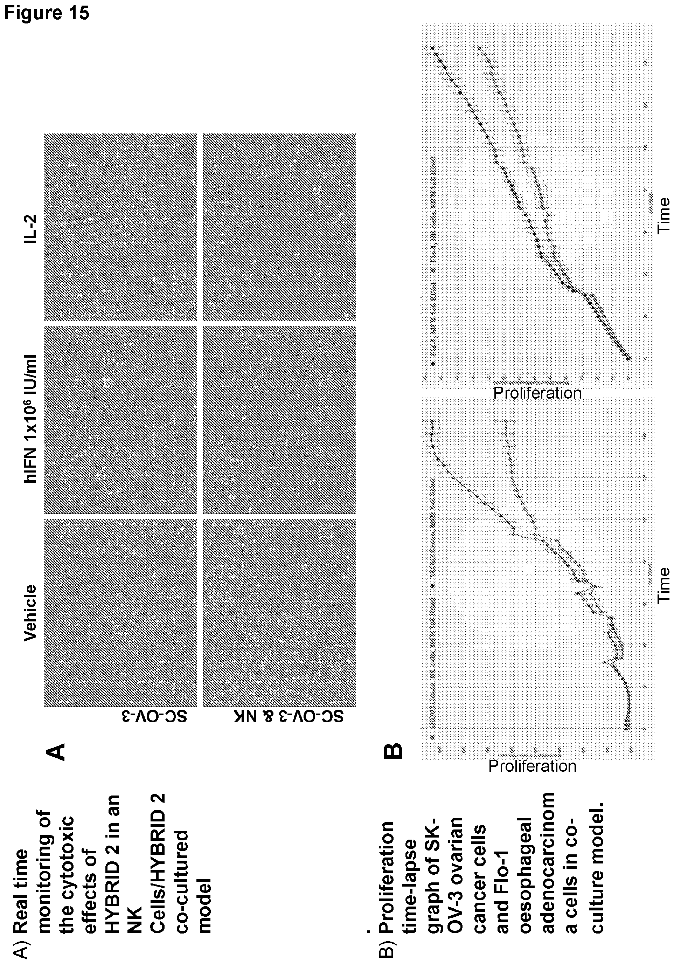

As indicated in the Figures 5 to 15, IFN alpha 14 and in particular Hybrid 2 has been demonstrated to modulate Il-17 (inhibits both 17 A and F very strongly), Il-6, CCL-5, and CCl-2 and to provide an antiviral NK response.

## Claims

1. An interferon alpha subtype, wherein the interferon alpha subtype is HYBRID 2 or a functionally active fragment of SEQ ID NO: 3 for use in the treatment and/or prophylaxis of a viral infection; wherein the viral infection is coronavirus infection and wherein HYBRID 2 comprises or consists of an amino acid sequence SEQ ID NO: 3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

2. The interferon alpha subtype for use in the treatment and/or prophylaxis of a viral infection of claim 1, wherein the coronavirus is Severe Acute Respiratory Syndrome Coronavirus-1 (SARS-CoV-1), Human Coronavirus NL63, Human Coronavirus HKU1, Middle East Respiratory Syndrome Coronavirus (MERS-CoV) or Severe Acute Respiratory Syndrome Coronavirus-2 (SARS-CoV-2 or Covid19).

3. The interferon alpha subtype for use in the treatment and/or prophylaxis of a viral infection of any one of claims 1 to 2, wherein the interferon alpha subtype is administered by sublingual, oral or injection administration.

4. The interferon subtype of claim 3, wherein the interferon alpha subtype is administered at a low dose of between 10⁴ to 5×10⁶ IU per day.

5. A composition comprising an interferon alpha subtype, wherein the interferon alpha subtype is HYBRID 2 or a functionally active fragment of SEQ ID NO:3, for use in the treatment and/or prophylaxis of a coronavirus infection wherein HYBRID 2 comprises or consists of an amino acid sequence SEQ ID NO: 3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

6. The composition of claim 5, further comprising an anti-viral compound.

7. The composition of claim 6, wherein the anti-viral compound is Remdesivir, LAM-002A, dexamethasone or Avigan.

## Patentansprüche

1. Ein Interferon-alpha-Subtyp, wobei der Interferon-alpha-Subtyp HYBRID 2 oder ein funktionell aktives Fragment von SEQ ID NO: 3 zur Verwendung bei der Behandlung und/oder Prophylaxe einer Virusinfektion ist; wobei die Virusinfektion eine Infektion mit dem Coronavirus ist und wobei HYBRID 2 eine folgende Aminosäuresequenz SEQ ID NO: 3 beinhaltet oder aus dieser besteht

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

2. Interferon-alpha-Subtyp zur Verwendung bei der Behandlung und/oder Prophylaxe einer Virusinfektion gemäß Anspruch 1, wobei das Coronavirus Schweres Akutes Respiratorisches Syndrom Coronavirus-1 (SARS-CoV-1), humanes Coronavirus NL63, humanes Coronavirus HKU1, Nahost-Atemwegssyndrom-Coronavirus (MERS-CoV) oder Schweres Akutes Respiratorisches Syndrom Coronavirus-2 (SARS-CoV-2 oder Covid19) ist.

3. Interferon-alpha-Subtyp zur Verwendung bei der Behandlung und/oder Prophylaxe einer Virusinfektion gemäß einem der Ansprüche 1 bis 2, wobei der Interferon-alpha-Subtyp durch sublinguale, orale oder Injektionsverabreichung verabreicht wird.

4. Interferon-Subtyp gemäß Anspruch 3, wobei der Interferon-alpha-Subtyp mit einer niedrigen Dosis von zwischen 10⁴ bis 5×10⁶ IE pro Tag verabreicht wird.

5. Eine Zusammensetzung, die einen Interferon-alpha-Subtyp beinhaltet, wobei der Interferon-alpha-Subtyp HYBRID 2 oder ein funktionell aktives Fragment von SEQ ID NO: 3 zur Verwendung bei der Behandlung und/oder Prophylaxe einer Infektion mit dem Coronavirus ist, wobei HYBRID 2 eine folgende Aminosäuresequenz SEQ ID NO: 3 beinhaltet oder aus dieser besteht

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

6. Zusammensetzung gemäß Anspruch 5, die ferner eine antivirale Verbindung beinhaltet.

7. Zusammensetzung gemäß Anspruch 6, wobei die antivirale Verbindung Remdesivir, LAM-002A, Dexamethason oder Avigan ist.

## Revendications

1. Un sous-type d'interféron alpha, le sous-type d'interféron alpha étant HYBRID 2 ou un fragment fonctionnellement actif de SEQ ID NO : 3 pour son utilisation dans le traitement et/ou la prophylaxie d'une infection virale ; où l'infection virale est une infection à coronavirus et où HYBRID 2 comprend ou consiste en une séquence d'acides aminés SEQ ID NO : 3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

2. Le sous-type d'interféron alpha pour son utilisation dans le traitement et/ou la prophylaxie d'une infection virale de la revendication 1, où le coronavirus est le Coronavirus-1 du syndrome respiratoire aigu sévère (SRAS-CoV-1), le Coronavirus humain NL63, le Coronavirus humain HKU1, le Coronavirus du syndrome respiratoire de Moyen-Orient (MERS-CoV) ou le Coronavirus-2 du syndrome respiratoire aigu sévère (SRAS-CoV-2 ou Covid19).

3. Le sous-type d'interféron alpha pour son utilisation dans le traitement et/ou la prophylaxie d'une infection virale de l'une quelconque des revendications 1 à 2, où le sous-type d'interféron alpha est administré par voie sublinguale, orale ou parentérale.

4. Le sous-type d'interféron de la revendication 3, le sous-type d'interféron alpha étant administré à une faible dose comprise entre 10⁴ et 5×10⁶ UI par jour.

5. Une composition comprenant un sous-type d'interféron alpha, le sous-type d'interféron alpha étant HYBRID 2 ou un fragment fonctionnellement actif de SEQ ID NO : 3, pour son utilisation dans le traitement et/ou la prophylaxie d'une infection à coronavirus où HYBRID 2 comprend ou consiste en une séquence d'acides aminés SEQ ID NO : 3

CDLPQTHSLGNRRALILLGQMGRISPFSCLKDRHDFRIPQEEFDGNQFQKAQAISVLHEM
MQQTFNLFSTKNSSAAWDETLLEKFYIELFQQMNDLEACVIQEVGVEETPLMNEDSILAV
KKYFQRITLYLIERKYSPCAWEVVRAEIMRSLSFSTNLQKRLRRKD.

6. La composition de la revendication 5, comprenant en sus un composé antiviral.

7. La composition de la revendication 6, où le composé antiviral est le remdésivir, le LAM-002A, la dexaméthasone ou l'Avigan.
